(19) **Europäisches Patentamt**
**European Patent Office**
**Office européen des brevets**

(11) Veröffentlichungsnummer : **0 531 255 A2**

(12) **EUROPÄISCHE PATENTANMELDUNG**

(21) Anmeldenummer : **92810637.6**

(22) Anmeldetag : **20.08.92**

(51) Int. Cl.⁵ : **B01J 19/32,** C02F 3/10, C12M 1/40

(30) Priorität : **21.08.91 CH 2451/91**

(43) Veröffentlichungstag der Anmeldung :
**10.03.93 Patentblatt 93/10**

(84) Benannte Vertragsstaaten :
**AT BE DE FR GB IT NL**

(71) Anmelder : **Knieza, Oleg**
**Oberemattstrasse 50 A**
**CH-4133 Pratteln (CH)**

(72) Erfinder : **Knieza, Oleg**
**Oberemattstrasse 50 A**
**CH-4133 Pratteln (CH)**

(54) **Packungselemente für den Stoff- und/oder Wärmeaustausch.**

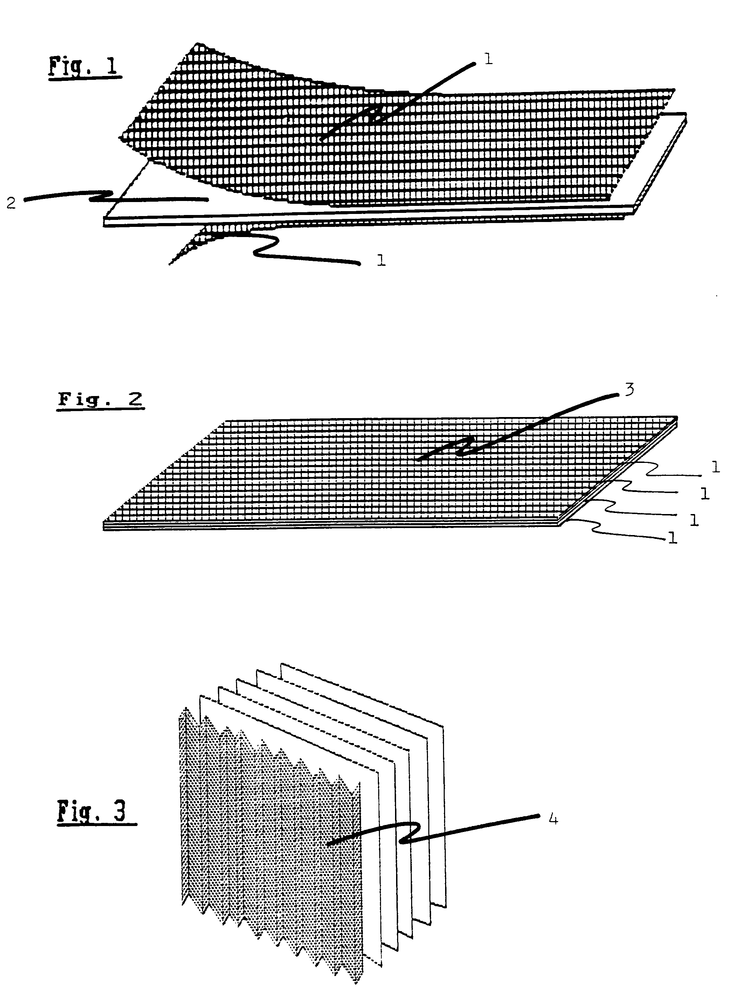

(57)    Eine Packungselemente (4) enthaltende Kolonne für den Stoffaustausch und/oder Wärmeaustausch zwischen einer gasförmigen, mindestens einer flüssigen und und mindestens einer festen Phase wird beschrieben. Die Packungselemente bestehen in der Compositbauweise aus einem Substrat (2), das auf seiner Oberfläche mit Fasern, Wolle oder Flächengebilden aus Glas und/oder Basalt beschichtet wird. Die Packungselemente in der Monoblockbauweise bestehen aus mehreren Glas- und/oder Basalt- und/oder Kohlenfasergewebeschichten, die miteinander verklebt sind (3).
Die Kolonne kann zum Destillieren, Rektifizieren, Absorbieren, Extrahieren oder zum Durchführen chemischer Reaktionen verwendet werden. (Fig. 1 bis 3)
Ein mit Packungselementen in Monoblockbauweise gefüllter durchströmter Behälter kann sehr wirksam als Biofilter eingesetzt werden.

Fig. 1

EP 0 531 255 A2

Die Erfindung betrifft eine Packungselemente enthaltende Kolonne für den Stoffaustausch zwischen einer gasförmigen, mindestens einer flüssigen Phase und mindestens einer festen Phase.

Der Erfindung liegt die Aufgabe zugrunde für den Einbau in Kolonnen katalytisch wirksame Packungselemente zu schaffen, welche thermisch, mechanisch und besonders in oxidierenden Medien chemisch stabil sind. Die katalytische Aktivität soll ohne Einbusse in allen Medien, stoff- und kolonnenunabhängig, voll wirksam sein.

Es sind Packungen oder Füllkörper für den Einbau in Stoffaustausch- und/oder Wärmeaustauschkolonnen bekannt, deren Aufgabe lediglich darin besteht die Austauschfläche für die miteinander im Kontakt stehenden Phasen zu bilden. Solche Packungselemente besitzen eine passive Oberfläche und können nicht aktiv am Stoff- und/oder Wärmeaustausch teilnehmen.

Es ist bekannt, dass Molekel von den Festkörpern an deren Oberflächen adsorbiert werden können. Die Adsorption, das bedeutet, die reversible Anlagerung von Gasen oder Flüssigkeiten an den Phasengrenzflächen, nämlich der Oberfläche eines festen Stoffes unter der Wirkung von van der Waals'schen oder elektrostatischen Kräften.

Es sind bereits aktive Packungen für den Einbau in Kolonnen bekannt, welche eine adsorptive Oberfläche aufweisen. Dadurch können solche Packungen aktiv am Stoff- und/oder Wärmeaustausch teilnehmen und die Trennleistung gegenüber den passiven Packungen, auf gleiches Kolonnenvolumen bezogen, um einen Faktor 2 bis 3 erhöhen.

Ferner ist bekannt und es gilt allgemein, wenn eine der vorhandenen Phasen im flüssigen Zustand vorliegt, so findet der Stoff- und/oder Wärmeaustausch nur an der benetzten Packungsoberfläche statt, die nicht benetzte Packungsoberfläche ist somit für den Stoff- und/oder Wärmeaustausch verloren. Alle bislang bekannten aktiven Packungen besitzen eine Oberfläche die entweder karbonisiert ist oder aus Kohlenstoff oder Kohlenstoffgewebe besteht.

Alle Packungen deren Stoffaustauschaktivität auf Kohlenstoff basiert, sind durch zwei starke, konzeptionell bedingte Nachteile in ihrer Anwendung und Leistung limitiert. Erstens:sie sind in oxidierenden Medien chemisch nicht stabil. In Anwesenheit von atomaren Sauerstoff oder sauerstoffenthaltenden Radikalen wird der Kohlenstoff zum Kohlenstoffdioxid oxidert. Besteht die Packung ganz aus Kohlenstoff, so bewirkt die oxidative Umwandlung des Kohlenstoffs innerhalb weniger Tage einen vollständigen Packungsverlust.

Besteht die aktive Packung aus einem mit Kohlenstoff-Fasern beschichteten Substrat, so geht die Stoffaustauschaktivität noch viel schneller verloren und die Trennleistung wird auf jene der passiven Packungen reduziert. Die Oxidationsgeschwindigkei ist temperaturabhängig und nimmt mit steigender Temperatur zu.

Zweitens:die Temperaturbeständigkeit der aktiven Packungen, deren Stoffaustauschaktivität auf Kohlenstoff basiert liegt in einer sauerstoffenthaltenden Gasphase bei etwa 300 °C.

Die erfindungsgemässe Packung ist dadurch gekennzeichnet, dass die Stoff- und/oder Wärmeaustauschaktivität mittels mineralischer Fasern gebildet wird. Je nach Anwendungsfall wird die Packung in der Compositbauweise oder als Monoblock hergestellt. In der Compositbauweise bestehen die Packungselemente aus einem beidseitig mit Glas- oder Basaltfasern beschichteten Substrat. Das Substrat kann aus Metall, keramischen Material oder Kunststoff, vorzugsweise thermoplastischen Kunststoff, insbesondere Polypropylen, Polyethylen, Polyvinylchlorid oder Polyvinylidenfluorid, bestehen. Es kann mit Gewebe, Vlies, Filz, Fasern, Wolle aus Glas oder Basalt beschichtet sein. Die Fertigung kann durch Verkleben oder Verschweissen der Glas- oder Basaltbeschichtung mit dem Substrat oder Aufwalzen, Aufpressen oder Thermokalandrieren der Glas- oder Basaltbeschichtung auf das Substrat erfolgen, wobei das Substrat vorher oder nachher in eine geeignete Form gebracht wird. Es eignen sich die üblichen Formen von Packungselementen für Kolonnen für den Stoffaustausch und/oder Wärmeaustausch.

Als Monoblock, das heisst in Monoblockbauweise, bestehen die Packungselemente aus Glas und/oder Basalt vorzugsweise aus mehreren aufeinander geschichteten, verklebten und definitiv ausgeformten Gewebe- oder Fasermatten.

Die in Monoblockbauweise hergestellten Packungselemente sind ohne Leistungsminderung auch in oxidierenden Medien bis zu einer Temperatur von 700°C thermisch und mechanisch stabil. Versuche haben ergeben, dass die Oberfläche der Glas- oder Basaltfaserbeschichtung, als Composit oder Monoblock, infolge der Kapillarkräfte in den Fasern resp. Filamenten von Flüssigkeiten aller Art, wie Wasser, wässrigen Lösungen organischen Lösemitteln oder schweren viskosen Ölen vollständig benetzt wird. Der Kapillardruck beträgt zwischen 1,5 bis 5 bar.

In der beiliegenden Zeichnung ist ein Ausführungsbeispiel des Erfindungsgegenstandes in der Composit- und Monoblockbauweise veranschaulicht. Es zeigen:

Fig. 1 ein beidseitig mit Glas- oder Basaltfasergewebe beschichtetes Substrat.

Fig. 2 ein Packungselement in Monoblockbauweise vor der Verformung.

Fig. 3 ein erfindugsgemässes Packungselement als Composit oder Monoblock.

Fig. 4 ein Diagramm, in dem die Trennleistung einer erfindungsgemässen Packung mit einer passiven, geordneten Packung gleicher spezifischen Oberfläche verglichen wird.

In Fig. 1 ist ein Substrat 2 dargestellt, das eine Dicke von 2 bis 3 mm hat und aus einem Thermoplasten besteht. Das Substrat ist beidseitig mit einem Glas- oder Basalt-Fasergewebematten 1 beschichtet.

Die Fig. 2 zeigt eine noch ungeformte Monoblockplatte 3. Sie besteht aus vier miteinander verklebten Glas- oder Basalt-Fasergewebematten 1 und hat eine Dicke von 2 bis 3 mm.

In Fig. 3 ist das ausgeformte Packungselement mit 4 bezeichnet.

Eine Absorptionskolonne, die erfindungsgemässe Packungselemente enthielt, wurde mit einer Absorptionskolonne verglichen, die die ältesten bekannten, passiven geordneten Packungen aus unbeschichteten Polypropylen enthielt.

Die erfindungsgemässen Packungen und die bekannten Packungen hatten die gleiche spezifische Oberfläche von 245 m²/m3 sowie die gleiche geometrische Form.

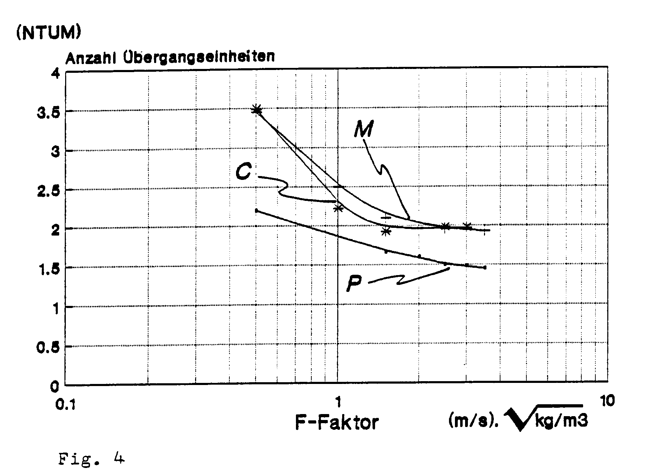

Das Ergebnis des Leistungsvergleichs ist in Fig. 4 wiedergegeben. Auf der Ordinate ist die Trennleistung, ausgedrückt als Anzahl der Übergangseinheiten/m (number of transfer units per meter, NTUM), aufgetragen und auf der Abszisse ist der Gasbelastungsfaktor $F = w . \sqrt{\delta}$, wobei w die auf den Kolonnenquerschnitt bezogene Gasgeschwindigkeit und $\delta$ die Gasdichte ist, ausgedrückt in $(m/s).\sqrt{kg/m3}$, aufgetragen. Die mit C bezeichnete Kurve zeigt die Werte der erfindungsgemässen Packung in Compositbauweise, mit M bezeichnete Kurve zeigt die Werte der erfindungsgemässen Packung in Monoblockbauweise und die mit P bezeichnete Kurve diejenige für eine passive Packung.

Der Kolonne wurde ein Gemisch aus Chlordioxid und Luft zugeführt, wobei die Eintrittskonzentration des C102 bei 1 bis 5 g/Nm3 lag. Das Gas wurde mit Wasser bei einer Berieselungsdichte von 5 bis 20 m3/m².h gewaschen. Die Betriebsbedingungen lagen bei allen Versuchen bei 20 °C Temperatur und 1 bar absolut. Die Trennleistung wurde nach den Formeln von Chilton und Colburn (Ind.Eng.Chem.27,1935) berechnet und ausgewertet. Die erfindungsgemässe Packung wies gegenüber einer Kolonne, die mit passiven Packungselementen bestückt war eine Verbesserung der Trennleistung um bis zu 100% auf.

Ein Leistungsvergleich mit einer aktiven Packung, auf der Basis von kohlenstoff, war wegen Kohlenstoffzerstörung infolge der Oxidation nicht möglich.

**Patentansprüche**

1. Packungselemente enthaltende Kolonne für den Stoffaustausch und/oder Wärmeaustausch zwischen einer gasförmigen, mindestens einer flüssigen und mindestens einer festen Phase, dadurch gekennzeichnet, dass die Packungselemente aus einem mindestens auf einem Teil seiner Oberfläche mit Fasern, Flächengebilden aus Glas- und/oder Basalt beschichteten Substrat bestehen.

2. Packungselemente enthaltender, durchströmter Behälter für die biologische Stoffzersetzung, dadurch gekennzeichnet, dass die Packungselemente vollständig, substratfrei aus Glas- und-/oder Basalt und/oder Kohlenstoff bestehen.

3. Kolonne nach Anspruch 1, dadurch gekennzeichnet, dass das Substrat aus Metall oder extrudierbaren Kunststoff, vorzugsweise Thermoplasten, insbesondere Polyethylen, Polypropylen, Polyvinylchlorid, Polyvinylidenfluorid, besteht.

4. Kolonne nach Anspruch 1 oder 3 dadurch gekennzeichnet, dass das Substrat mit Gewebe, Wolle, Fasern aus Glas und/oder Basalt beschichtet ist.

5. Kolonne nach Anspruch 1,3 oder 4 dadurch gekennzeichnet, dass die Beschichtung mit dem Substrat verklebt oder verschweisst, auf das Substrat aufgewalzt oder thermokalandriert ist.

6. Behälter nach Anspruch 2 dadurch gekennzeichnet, dass die Packungselemente substratfrei aus miteinander verklebten Glas- und/oder Basaltasern und/oder Kohlenstofffasern bestehen.

7. Verwendung einer Packungselemente enthalten Kolonne nach einem der Ansprüche 1 bis 6 zum Destillieren, Rektifizieren, Absorbieren, Extrahieren oder zum Durchführen chemischer Reaktionen.

8. Verwendung eines Packungselemente enthaltenden Behälters nach Anspruch 2 als Biofilter.

9. Verwendung der Packungselemente nach Anspruch 2 als immobilisierungsgeeigneter Träger mikrobiologischer Kulturen.

Fig. 1

Fig. 2

Fig. 3

Fig. 4